# EUROPÄISCHE PATENTSCHRIFT

(11) **EP 3 426 054 B1**
(45) Veröffentlichungstag und Bekanntmachung des Hinweises auf die Patenterteilung: **13.10.2021**
(21) Anmeldenummer: 17714391.4
(22) Anmeldetag: 03.03.2017
(51) Int. Cl.: A23G 3/56, A43D 3/14, A61B 13/00, B31C 5/00, B31D 5/00

(54) **EISSTIEL GEBILDET AUS EINEM PAPIERPROFIL**
ICE STICK MADE FROM A PAPER PROFILE
BÂTONNET DE GLACE FORMÉ Á PARTIR D'UN PROFILÉ EN PAPIER

(30) Priorität: 07.03.2016 DE 202016001413 U
(43) Veröffentlichungstag der Anmeldung: 16.01.2019
(73) Patentinhaber: Setter GmbH&Co. Papierverarbeitung, 46446 Emmerich (DE)
(72) Erfinder: GRASSE, Steffen, 46446 Emmerich (DE); HÜLKENBERG, Roland, 46446 Emmerich (DE); WINS, Leo, 46446 Emmerich (DE); JOCHEM, Franz, 46446 Emmerich (DE)
(74) Vertreter: Grosse Schumacher Knauer von Hirschhausen
(86) Internationale Anmeldenummer: PCT/EP2017/000286
(87) Internationale Veröffentlichungsnummer: WO 2017/153039

(56) Entgegenhaltungen:
- WO-A1-93/13279
- DE-C- 896 997
- DE-U1- 9 205 257
- FR-A- 335 951
- FR-A1- 2 787 009
- GB-A- 880 627
- GB-A- 903 317
- US-A- 2 255 887
- US-A- 2 369 006
- US-A1- 2004 131 825
- US-B1- 6 192 540

## Beschreibung

### GEBIET DER ERFINDUNG

Die Erfindung betrifft einen Eisstiel für Speiseeis gebildet aus einem Papierprofil nach dem Oberbegriff des Anspruchs 1. Das Papierprofil weist eine längliche Form auf. Unter einem "Profil" wird hier ein längliches Bauteil mit unbestimmtem Querschnitt verstanden, welches etwa eine breitflache Form aufweisen kann. Das Profil umfasst zumindest eine in Längsrichtung verlaufende Längsseite, zumindest eine in Querrichtung verlaufende Schmalseite und als flaches Profil eine in Querrichtung verlaufende Profildicke oder -stärke.

### TECHNOLOGISCHER HINTERGRUND

In der (Lebensmittel-)Verpackungsindustrie, in der Hygieneartikelherstellung und bei der Fertigung medizinischer oder orthopädischer Hilfsmittel wird als Werkstoff regelmäßig Holz verwendet. Holz ist zwar biologisch abbaubar und oder recyclefähig, jedoch vermag Holz zu brechen und dabei zu splittern. Außerdem lässt sich Holz als Werkstoff nur eingeschränkt verarbeiten. So kann Holz zum Beispiel nur mit beschränkten, in der Verpackungsindustrie zulässigen, Möglichkeiten farbig gestaltet werden, insbesondere sofern der Holzwerkstoff direkt mit einem Lebensmittel in Kontakt tritt. Ein Papierwerkstoff wird in der US 2 255 887 A beschrieben.

Es wurden daher Anstrengungen unternommen, anstelle eines Holzwerkstoffs einen Kunststoffwerkstoff industriell zu verarbeiten. Kunststoffe jedoch haben den erheblichen Nachteil, dass sie biologisch nicht oder mit vergleichsweise großem Aufwand abbaubar sind, und dass sowohl Herstellung, wie auch Verarbeitung und Entsorgung zu höheren Kosten führen. Wenn ein Kunststoffwerkstoff bricht, entsteht zudem oft eine scharfe Kante, die bei Verpackungen oder in Zusammenhang mit medizinischen Zwecken schädlich ist. Außerdem enthalten Kunststoffe oftmals ungesunde Bestandteile, wodurch in gesundheitlicher Hinsicht Gefahren drohen. Insofern ist Kunststoff in vielerlei Hinsicht keine geeignete Alternative zum Holzwerkstoff.

### DARSTELLUNG DER ERFINDUNG

Vor diesem Hintergrund besteht die Aufgabe darin, technische Maßnahmen anzugeben, mit denen die Nachteile des Holzwerkstoffs, insbesondere in ökologischer Hinsicht, unter Vermeidung einer Verwendung von Kunststoffen beseitigt werden. Diese Aufgabe wird durch einen Eisstiel nach Anspruch 1 gelöst. Ausgestaltungen und/oder Varianten der Erfindung, die die hierin genannte Aufgabe lösen, ergeben sich aus den abhängigen Ansprüchen. Das Papierprofil für den Eisstiel gemäß Anspruch 1 wäre für ein Verpackungselement, Verpackungsmittel, und/oder eine Verpackungsausrüstung, bevorzugt jeweils einer Lebensmittelverpackung geeignet.

Das Papierprofil ist lebensmittelverträglich, was etwa dadurch erreichbar ist, dass das Papierprofil respektive jeder Papierprofilbestandteil nur Papier umfasst und nicht etwa einen (Industrie- )Klebstoff. Insofern besteht das Papierprofil bevorzugt ausschließlich aus Zellstoff. Das Papierprofil könnte ferner für Zwecke im medizinischen, hygienischen, orthopädischen und/oder in anderen Konsumbereichen vorgesehen sein. Das Papierprofil weist eine längliche Form auf und ist aus mehreren runden oder nahezu runden biegesteifen Papierstäben gebildet. Jeder Papierstab ist bevorzugt aus einer Papierbahn gebildet, als Haftvermittler kann Wasser vorgesehen sein. Der Papierstab umfasst bevorzugt keinen Hohlraum und ist insofern ein solider Papierstab. Ein Papierprofil, das aus einem soliden Papierstab gebildet ist, ist insofern ein solides, d. h. hohlraumfreies, Papierprofil. Ein Profil, das aus mehreren Stäben gebildet ist, kann als ein solides respektive im Wesentlichen solides Profil gebildet sein. Der Durchmesser eines Papierstabs des Papierprofils liegt in einem Durchmesserbereich von etwa 1 mm bis etwa 12 mm, beispielsweise beträgt der Durchmesser etwa 2 mm oder etwa 3 mm. Bevorzugt kann durch Biegen des Papierprofils zumindest bereichsweise eine rückstellende, der Biegerichtung entgegenwirkende, Kraft, d. h. eine Rückstellkraft erzeugbar sein. Es kann auch eine eine Vorspannung erzeugende, insbesondere eine etwaige Rückstellkraft beeinflussende, Kraftwirkung des Papierprofils vorgesehen sein, die insbesondere durch mehrere, zumindest abschnittsweise miteinander verbundene Papierstäbe erzeugbar ist. Die Form (Querschnitt) und/oder Dichte der Papierstäbe kann durch Bearbeitung der Stäbe beim Herstellen des Profils verändert worden sein, etwa durch Pressen oder Quetschen. Die Biegesteifigkeit des Papierprofils entspricht in zumindest einer Biegerichtung zumindest der Biegesteifigkeit eines der biegesteifen Papierstäbe. Jeder biegesteife Papierstab des Papierprofils umfasst eine Biegesteifigkeit, die mehr als das etwa 2-fache, insbesondere das etwa 5-fache bis etwa 150-fache, und bevorzugt das etwa 100-fache, der Biegesteifigkeit eines ähnlich dimensionierten Papier- oder Pappkörpers insbesondere der Biegesteifigkeit eines Körpers, der nicht aus einer Papierbahn gebildet/gefertigt ist, aufweist.

Unter einem Stab wir hier ein länglicher Gegenstand mit einer zylindrischen oder nahezu zylindrischen Form verstanden. Der Papierstab ist aus Papier gebildet, wobei auch ein dünner Karton respektive eine dünne Kartonbahn zur Fertigung des Papierstabs geeignet sein kann. Papier umfasst ein holzstoff- und/oder zellulosehaltiges Ausgangsmaterial, d. h. ein Material das im Wesentlichen auf abbaubaren Pflanzenfasern basiert, welches zu einem biegesteifen (Rund-)Stab, dem Papierstab, verarbeitet ist. Biegesteif meint hier, dass der Papierstab - verglichen mit Holz und/oder Kunststoff - mit mechanischen Kräften/Momenten beaufschlagt werden kann, ohne dabei zu brechen oder zu splittern oder unter Bildung scharfer Kanten zu knicken oder zu reißen. Etwaige Verletzungsgefahren im Zusammenhang mit der Verwendung des Profils sind daher gegenüber den Alternativen Holz oder Kunststoff reduziert. Werkstoffkundlich betrachtet scheint Papier zwar weicher oder flexibler zu sein als Holz/Kunststoff, jedoch in der Form/Gestalt als hierin beschriebener Papierstab hat das Material eine hohe Festigkeit/Stabilität sowie eine hohe Biegesteifigkeit. Diese Eigenschaften des Papierstabs charakterisieren die Eigenschaften des Papierprofils, wodurch das Profil den geforderten mechanischen und/oder werkstofflichen Anforderungen (auch bei unterschiedlichen Einsatzzwecken) genügt.

Ein besonderer Vorteil ergibt sich dann, wenn das Papierprofil in Kontakt mit Lebensmitteln treten soll, etwa im Zusammenhang mit einer Lebensmittelverpackung: Papier als Werkstoff genügt in vielerlei Hinsicht einer Vielzahl lebensmittelrechtlicher Anforderungen und kann ohne besonderen Aufwand unmittelbar verwendet und verarbeitet werden. Insofern ist auch das Profil, welches aus mehreren (soliden) Papierstäben gebildet ist, für einen Lebensmittelkontakt unmittelbar geeignet. Hinsichtlich ökologischer Gesichtspunkte lässt sich ein hierin beschriebenes Papierprofil insofern gut entsorgen, als es abbaubar ist und mit dem Papiermüll ("Papiertonne") entsorgbar ist. Eine gesonderte Entsorgung, etwa mit dem Restmüll ("Restmülltonne"), wie es etwa bei einem Holzprofil oder Holzstab der Fall wäre, entfällt bei dem Papierprofil.

Je nach Verarbeitung des Papierstabs zur Bildung des erfindungsgemäßen Eisstiels ist vorgesehen, dass die Biegesteifigkeit des Papierprofils die Biegesteifigkeit eines der Papierstäbe in zumindest einer Biegerichtung übertrifft.

Gemäß einer bevorzugten Ausgestaltung des Profils kann vorgesehen sein, dass zumindest einer der Papierstäbe durch Pressen respektive Flachdrücken oder Quetschen bearbeitet ist, wodurch eine Variante des (soliden) Papierprofils flach ist. Flachdrücken meint, dass der ursprünglich zylindrische oder nahezu zylindrische Papierstab mit kreisrundem oder nahezu kreisrundem Querschnitt mechanisch so bearbeitet wird, dass sein Querschnitt oval oder nahezu oval oder an zumindest einer Seite oder an zwei gegenüberliegenden Seiten flach ist. Das daraus gebildete Profil ist flach, d. h. die Dicke ist kleiner als die Abmessung der Schmalseite. Bei dieser Variante hat das Profil parallel zur Schmalseite des Profils, also quer zur Dicke des Profils, eine Biegesteifigkeit, die höher ist als die Biegesteifigkeit des zylindrischen Papierstabs, aus dem das Profil gebildet ist.

Bevorzugt kann das (im Wesentlichen solide) Papierprofil aus zumindest einer Lage Papierstäben gebildet sein. In dieser Lage Papierstäbe (Papierstab-Platte) sind zumindest zwei Papierstäbe nebeneinander und zueinander parallel angeordnet. Wahlweise können auch drei, vier, fünf oder mehr Papierstäbe innerhalb einer Lage nebeneinander angeordnet sein, welche ein flaches Papierprofil bilden. Auch hier ist die Biegesteifigkeit parallel zur Orientierung der Papierstäbe-Lage größer als quer dazu. In der durch die Profildicke bestimmten Orientierung ist die Biegesteifigkeit des Profils im Wesentlichen durch die eines der Stäbe charakterisiert.

Bei mehreren in einer Lage angeordneten Stäben wird die Stabilität des Profils in zumindest einer Richtung erhöht. Soll auch eine Verbesserung der Stabilität in einer zweiten Richtung erfolgen, können mehrere Lagen Papierstäbe aufeinander angeordnet werden. Das Papierprofil kann demnach mehrlagig aus Papierstäben gebildet sein, wobei auf einer ersten Lage Papierstäbe zumindest eine zweite Lage Papierstäbe angeordnet ist. Die Zentren der Papierstäbe benachbarter Papierstab-Lagen können bezogen auf den Profilquerschnitt übereinander angeordnet sein oder es kann ein Versatz dieser Zentren vorgesehen sein.

Bei der Ausgestaltung des Papierprofils mit einer oder mehreren Lagen Papierstäben (Papierstab-Gitterwerk, Papierstab-Verband, Papierstab-Fachwerk) können Rillen auf der Oberfläche des Profils vorgesehen sein. Die Rillen können die Haptik des Profils verbessern, sie können auch ein Gestaltungsbeitrag sein, etwa als Erkennungsmerkmal, beispielsweise um das Profil optisch und/oder haptisch von anderen Profilen zu unterscheiden.

Sofern das Profil eine glatte, d. h. im Wesentlichen rillenfreie, Oberfläche haben soll, kann eine Bearbeitung des Profils zur Beseitigung etwaiger Rillen vorgesehen sein. Die Rillen können verfüllt sein oder das Profil kann durch Pressen und/oder Schleifen und/oder Schneiden geglättet sein.

An zumindest einer der Längsseiten des Profils und/oder an zumindest einer der Schmalseiten des Profils kann (abschnittsweise) zumindest eine Rundung und/oder Schräge vorgesehen sein, insbesondere so, dass das Papierprofil eine Kontur aufweist. Die Kontur kann so gebildet sein, dass die Profilbreite entlang der Profillänge variiert. Bevorzugt kann das Profil die Kontur eines Paddels aufweisen. Zum einen wird das Profil dadurch besser handhabbar, da es an einem Ende oder an beiden Enden breiter ist. Zum anderen wird dadurch, dass das Profil an zumindest einer Stelle schmaler ist, Material eingespart, was einen biologischen Abbau beschleunigt.

Zur Bildung des erfinderischen Eisstiels ist vorgesehen, dass die Papierstäbe einen identischen oder nahezu identischen Durchmesser haben. Die Papierstäbe des Papierprofils können für nicht erfindungsgemäße Ausgestaltungen aus Papierstäben mit einem ersten Durchmesser und zumindest aus Papierstäben mit einem zweiten Durchmesser gebildet sein, wobei der erste Durchmesser kleiner ist als der zweite Durchmesser. Beispielsweise können innerhalb einer Papierstab-Lage Papierstäbe mit großem (kleinem) Durchmesser benachbart sein zu Papierstäben mit kleinem (großem) Durchmesser. Sofern eine zweite Lage entsprechende vorgesehen ist, können die Papierstäbe mit großem Durchmesser der ersten Lage benachbart sein zu Papierstäben mit kleinem Durchmesser der zweiten Lage. Diese Anordnung führt zu einer dichten Packung, in der das Volumen der Zwischenräume zwischen benachbarten Papierstäben innerhalb des Profils möglichst klein ist. Alternativ können auch mehr als zwei unterschiedliche Durchmesser vorgesehen sein, etwa ein erster Durchmesser, ein zweiter, ein dritter, ein vierter, usw..

Innerhalb einer Papierstab-Lage können Papierstäbe mit dem zweiten (größeren) Durchmesser durch Pressen respektive Flachdrücken bearbeitet sein, so dass die Variation der Dicke der Lage entlang der Breite der Lage möglichst gering ist. Die Lage wird dadurch flacher und weniger profiliert.

Es kann zweckmäßig sein, wenn sich zumindest ein Papierstab in einer Eigenschaft von zumindest einem anderen Papierstab unterscheidet, wobei die Eigenschaft Form, Rundung, Farbe, Oberflächenbeschaffenheit, Material und/oder Biegesteifigkeit umfassen kann. So kann ein Profil vorgesehen sein, welches aus zwei, drei, vier oder mehreren Papierstäben gebildet ist, die sich jeweils in ihrer Farbe unterscheiden. Das Profil wird dadurch mehrfarbig, ohne dass das Profil respektive zumindest ein Profilabschnitt nach Fertigung mittels Papierstäben eingefärbt werden muss. Es kann vorgesehen sein, dass der Anwender oder Käufer des Profils mitbestimmt, welche Farben die Stäbe aufweisen, und welche farbliche Gestalt das Profil aufweisen soll.

Hinsichtlich der Form können beispielsweise innerhalb einer Lage außen dünnere Stäbe angeordnet werden als innen, wodurch das Profil innen dicker ist als außen. Einige der Papierstäbe des Papierprofils können endseitig oder längsseitig abgerundet sein. Es kann sich dadurch eine Paddelform oder die Form eines Doppelpaddels bilden. Es kann ferner vorgesehen sein, dass einzelne oder sämtliche Stäbe eines Profils durch (Zusammen-)Schnüren, Schneiden und/oder Stanzen vorbehandelt sind, wodurch das Profil hinsichtlich Form und Stabilität bestimmte Eigenschaften erhält. Einzelne oder mehrere Stäbe können auch eine raue oder aufgeraute Papieroberfläche aufweisen. Es können auch Stäbe aus unterschiedlichen Papiersorten verarbeitet sein.

Damit die (im Wesentlichen soliden) Papierstäbe innerhalb eines Papierprofils stabil und fest, wahlweise unter Bildung einer Vorspannung, anordbar werden, kann vorgesehen sein, dass zumindest einige der Papierstäbe des Papierprofils, vorzugsweise sämtliche Papierstäbe des Papierprofils zumindest abschnittsweise, insbesondere durch Haften/Kleben/Leimen, untereinander und/oder miteinander verbunden sind. Als Haftvermittler kann Wasser verwendet werden und/oder ein insbesondere lebensmittelverträglicher Klebstoff/Leim. Es kann eine (Haft-)Verbindung entlang der Längsseite der Papierstäbe vorgesehen sein oder eine Verbindung an einer oder mehreren Verbindungsstellen. Die Verbindungsstelle kann am Stabende (endseitig) oder im Bereich der Stabmitte (mittig) sein. Sofern mehrere Verbindungsstellen oder -punkte vorgesehen sind, können diese etwa entlang der Längsrichtung des Stabs verteilt sein. Es kann alternativ oder kumulativ vorgesehen sein, dass die Papierstäbe entlang des Umfangs des Papierprofils durch zumindest ein, insbesondere aus Papier gebildetes, Mantelelement miteinander verbunden sind. Dieses Mantelelement kann als Papier-Banderole gebildet sein, die Papierbanderole kann einlagig oder mehrlagig gebildet sein. Durch einen (Papier-)Mantel wird erreicht, dass das Profil außen eine im Wesentlichen glatte Oberfläche aufweist. Das Mantelelement kann auch mehrteilig sein, etwa aus mehreren Mantelabschnitten, die entlang der Länge des Profils benachbart, ggf. mit Unterbrechungen, angeordnet sind. Das Papierprofil und/oder das Mantelelement kann eine insbesondere lebensmittelverträgliche Feuchtigkeitsbarriere umfassen, die etwa durch Beschichten oder Imprägnieren gebildet ist. Damit wird vermieden, dass Feuchtigkeit in die Profilbestandteile eindringt, was der Beständigkeit der Biegesteifigkeit des Profils zuträglich ist.

Es kann vorgesehen sein, dass auf dem Profil ein Kennzeichnungsbereich angeordnet ist, auf dem eine Kennzeichnung und/oder ein Logo anordbar ist. Der Kennzeichnungsbereich kann auf einem Papierstab, auf mehreren (benachbarten) Papierstäben oder auf einem um die Papierstäbe herum angeordneten Mantelelement vorgesehen sein. In dem Kennzeichnungsbereich können ein Firmenhinweis oder ein lebensmittelrechtlicher Hinweis, ein Verwendungshinweis und/oder ein sonstiger Hinweis, etwa als Beschriftung, angeordnet sein.

Technische Maßnahmen, mit denen Nachteile des Holzwerkstoffs, insbesondere in ökologischer Hinsicht, unter Vermeidung einer Verwendung von Kunststoffen beseitigt werden können, ergeben sich ferner durch ein Verfahren zum Herstellen eines Papierprofils aus Papierstäben, insbesondere eines hier beschriebenen Papierprofils. Demnach werden der oder die Papierstäbe zunächst angeordnet. Gemäß einem weiteren Verfahrensschritt werden der oder die Papierstäbe umgeformt und/oder miteinander verbunden. Das Umformen kann ein Pressen, insbesondere ein Flachpressen, umfassen. Das Verbinden kann Kleben, Leimen und/oder Siegeln umfassen. Es kann auch ein Beschichten mit einem oder mehreren Beschichtungsmitteln vorgesehen sein. Es kann in einem weiteren Verfahrensschritt vorgesehen sein, dass zumindest einer oder mehrere, vorzugsweise sämtliche, Papierstäbe bearbeitet werden, insbesondere durch Schneiden, Stanzen, Ablängen, Färben, Bedrucken, Umwickeln, Glätten, Formen, Abrunden, Aromatisieren, Imprägnieren und/oder Schneiden. Die Papierstäbe können aus Zellstoff gebildet sein/werden, d. h. aus dem Zellstoff wird ein Papiermaterial gebildet. Der Zellstoff kann in ökologisch nachhaltiger Weise etwa aus Bruchholz gewonnen werden, also nicht zwingend aus gewachsenem Holz, wie es etwa bei einem Holzprofil der Fall wäre.

Weitere technische Maßnahmen, mit denen die Nachteile des Holzwerkstoffs, insbesondere in ökologischer Hinsicht, unter Vermeidung einer Verwendung von Kunststoffen beseitigt werden, ergeben sich durch eine Verwendung eines Papierstabs oder eines Papierstabverbunds. Die Verwendung betrifft insbesondere ein hier beschriebenes Papierprofil, welches insbesondere nach einem hier beschriebenen Verfahren gebildet sein kann. Demnach wird das Papierprofil respektive der Papierstab oder ein Papierstabverbund, also ein Verbund mehrerer Papierstäbe, als Verpackungsmittel und/oder Verpackungselement verwendet. Das Verpackungsmittel respektive Verpackungselement oder die Komponente einer Verpackungsausrüstung ist insbesondere für Lebensmittelverpackungen geeignet. Da Papier biologisch besonders leicht abbaubar ist, entfällt ein aufwändiges Recycling der Verpackungskomponente. Das Verpackungselement kann dabei ein Hilfsmittel zum Entnehmen des Verpackungsinhalts betreffen, eine Art Verzehrwerkzeug als Verpackungsbeigabe (Picker, Griffelement). Es kann aber auch als mechanische Verstärkungskomponente für Papier-, Papp- oder Karton-Verpackungen vorgesehen sein. Das Verpackungsmittel kann auch als Fixierhilfe und/oder als Bewehrung, etwa zum Verstärken der Verpackung oder in der Verpackung zum Fixieren des Verpackungsinhalts vorgesehen sein.

Besonders bevorzugt ist die Verwendung des hier beschriebenen Profils als Griffelement oder Stiel für Süßwaren, insbesondere für Speiseeis. Bei Speiseeis "am Stiel" oder bei Stieleiscreme trifft die Besonderheit zu, dass das Lebensmittel in direktem Kontakt mit dem Eisstiel tritt: Der Eisstiel wird in das Speiseeis eingearbeitet und der aus dem Speiseeis herausragende Teil des Eisstiels dient als Griff zum bequemen Verzehr des Speiseeises. Indem der hier beschriebene Eisstiel aus dem hier beschriebenen Papierprofil gebildet ist, werden die Nachteile von Holz-Eisstielen überwunden. Bei starker mechanischer Beanspruchung wird der erfindungsgemäße Papierprofil-Eisstiel nicht brechen oder reißen und, insbesondere beim Knicken, nicht splittern. Sollte er in Folge einer starken mechanischen Beanspruchung knicken, wird die Knickstelle deutlich weniger oder gar nicht scharfkantig sein, als dies bei einer Bruch- oder Knickstelle bei einem Holz- oder Kunststoffwerkstoff der Fall wäre. Hinsichtlich der Entsorgung wurde bereits auf die Vorteile eines Papierwerkstoffs hingewiesen. Ein hier beschriebener Eisstiel aus einem hier beschriebenen Papierprofil kann, etwa zusammen mit der Papier-Umverpackung der Eiscreme, einheitlich als Papiermüll entsorgt werden. Eine Mülltrennung in Papiermüll (Umverpackung) und Restmüll (Stiel, beispielsweise aus Holz oder Kunststoff) ist nicht erforderlich. Hinsichtlich der Stabilität hat der hier beschriebene, aus einem hier beschriebenen Papierprofil gebildete, Eisstiel eine hinreichend hohe, insbesondere mit einem Kunststoffwerkstoff vergleichbare, Festigkeit. Die Biegesteifigkeit ist verglichen mit herkömmlichen Werkstoffen höher. Der erfindungsgemäße Stiel vermag das Speiseeis zu tragen, und verträgt eine robuste Handhabung durch den Anwender/Verbraucher, der den Eisstiel zum Verzehr des Speiseeises verwendet.

Es besteht die Möglichkeit, auf dem den Eisstiel bildenden Papierprofil Geschmacks- und/oder Geruchs-Aromen aufzulegen oder einzuarbeiten, so dass der Verbraucher ein zusätzliches Geschmacks- und/oder Geruchserlebnis beim Verzehr des Speiseeises erfahren kann. Eine Aromatisierung/Imprägnierung bei einem Kunststoffstiel ist nicht möglich und bei einem Holzstiel bestünde Verletzungsgefahr, insbesondere wenn etwa ein Kind versuchen würde, einen aromatisierten/imprägnierten Holzstiel zu zerkauen. Im Zusammenhang mit einem Stiel aus einem hierin beschriebenen Papierprofil können weitere organoleptische Möglichkeiten ausgeschöpft werden.

Weitere technische Maßnahmen, mit denen die Nachteile des Holzwerkstoffs, insbesondere in ökologischer Hinsicht, unter Vermeidung einer Verwendung von Kunststoffen beseitigt werden, ergeben sich durch eine Verwendung eines hier beschriebenen Papierstabs oder eines hier beschriebenen Papierstabverbunds. Demnach wird das Papierprofil als Hygienewerkzeug und/oder als, insbesondere medizinisches, Untersuchungsinstrument, vorzugsweise als Mundspatel oder Tupfer oder Tupfer-Halter verwendet. Neben den oben beschriebenen Vorteilen bezüglich verminderter Verletzungsgefahr in Folge verminderter Bruchgefahr bietet sich ferner die Möglichkeit das hier beschriebene Papierprofil als (Papier-)Spatel mit medizinisch relevanten Wirkstoffen anzureichern. Zusätzliche Hygiene-Vorkehrungen, die über das hinausgehen, was bei einem Holzspatel erforderlich wäre, entfallen.

Weitere technische Maßnahmen, mit denen die Nachteile des Holzwerkstoffs, insbesondere in ökologischer Hinsicht, unter Vermeidung einer Verwendung von Kunststoffen beseitigt werden, ergeben sich durch einen Eisstiel für Speiseeis.

Demnach ist der Eisstiel aus einem hier beschriebenen Papierprofil gebildet.

### KURZBESCHREIBUNG DER FIGUREN

In der Zeichnung zeigen
- Fig. 1: eine schematische Darstellung eines Papierprofils umfassend einen mechanisch geformten Papierstab in seitlicher Ansicht,
- Fig. 1a bis 1c: schematische Darstellungen von Papierprofilen, jeweils umfassend mehrere mechanisch geformte Papierstäbe, jeweils in seitlicher Ansicht,
- Fig. 2a bis 2c: schematische Darstellungen von Papierprofilen, jeweils umfassend mehrere Papierstäbe, jeweils in seitlicher Ansicht,
- Fig. 3a, 3b: schematische Darstellungen von Papierprofilen, jeweils umfassend mehrere Papierstäbe, teilweise mechanisch geformt, jeweils in seitlicher Ansicht,
- Fig. 4a bis 4d: schematische Darstellungen von Papierprofilen, jeweils umfassend mehrere Lagen mit jeweils mehreren flachgedrückten Papierstäben, jeweils in seitlicher Ansicht,
- Fig. 5a bis 5d: schematische Darstellungen von Papierprofilen, jeweils umfassend mehrere Lagen mit jeweils mehreren Papierstäben, jeweils in seitlicher Ansicht,
- Fig. 6b bis 6d: schematische Darstellungen von Papierprofilen, jeweils umfassend mehrere, zueinander versetzte Lagen mit jeweils einem oder mehreren Papierstäben, jeweils in seitlicher Ansicht
- Fig. 7: eine schematische Darstellung eines Papierprofils umfassend mehrere Lagen mit teilweise flachgedrückten Papierstäben, in seitlicher Ansicht,
- Fig. 8a bis 8c: schematische Darstellungen von Papierprofilen, jeweils umfassend eine umwickelte Lage mit mehreren flachgedrückten Papierstäben, jeweils in seitlicher Ansicht,
- Fig. 9a bis 9c: schematische Darstellungen von Papierprofilen, jeweils umfassend eine umwickelte Lage mit mehreren Papierstäben, jeweils in seitlicher Ansicht,
- Fig. 10a, 10b: schematische Darstellungen von Papierprofilen, jeweils umfassend eine umwickelte Lage mit mehreren, teilweise flachgedrückten, Papierstäben, jeweils in seitlicher Ansicht,
- Fig. 11a bis 11d: schematische Darstellungen von Papierprofilen, jeweils umfassend mehrere umwickelte Lagen mit jeweils einem oder mehreren flachgedrückten Papierstäben, jeweils in seitlicher Ansicht,
- Fig. 12a bis 12d: schematische Darstellungen von Papierprofilen, jeweils umfassend mehrere umwickelte Lagen mit jeweils einem oder mehreren Papierstäben, jeweils in seitlicher Ansicht,
- Fig. 13b bis 13d: schematische Darstellungen von Papierprofilen, jeweils umfassend mehrere umwickelte und versetzt angeordnete Lagen mit jeweils mehreren Papierstäben, jeweils in seitlicher Ansicht,
- Fig. 14: eine schematische Darstellung eines Papierprofils umfassend mehrere umwickelte Lagen mit jeweils mehreren teilweise flachgedrückten Papierstäben, jeweils in seitlicher Ansicht,
- Fig. 15: einen Papierstabverbund in perspektivischer Ansicht,
- Fig. 16a bis 16c: schematische Darstellungen von Papierprofilen mit mehreren Papierstäben für erfindungsgemäße Eisstiele, jeweils in Aufsicht,
- Fig. 17: eine schematische Darstellung eines Papierprofils mit einer Kontur in Aufsicht,
- Fig. 18a, 18b: schematische Darstellungen eines Speiseeis in seitlicher Ansicht,
- Fig. 19a, 19b: schematische Darstellungen von Eisstielen in perspektivischer Ansicht,
- Fig. 20: eine schematische Darstellung eines erfindungsgemäßen Eisstiels in perspektivischer Ansicht,
- Fig. 21: eine schematische Darstellung einer Variante eines Eisstiels in perspektivischer Ansicht,
- Fig. 22a: eine schematische Darstellung einer nicht erfindungsgemäßen Spannvorrichtung für einen Schuh in ungebogenem Zustand und
- Fig. 22b: eine schematische Darstellung einer nicht erfindungsgemäßen gebogenen Spannvorrichtung in einem Damenschuh.

### DETAILLIERTE BESCHREIBUNG VON AUSFÜHRUNGSBEISPIELEN

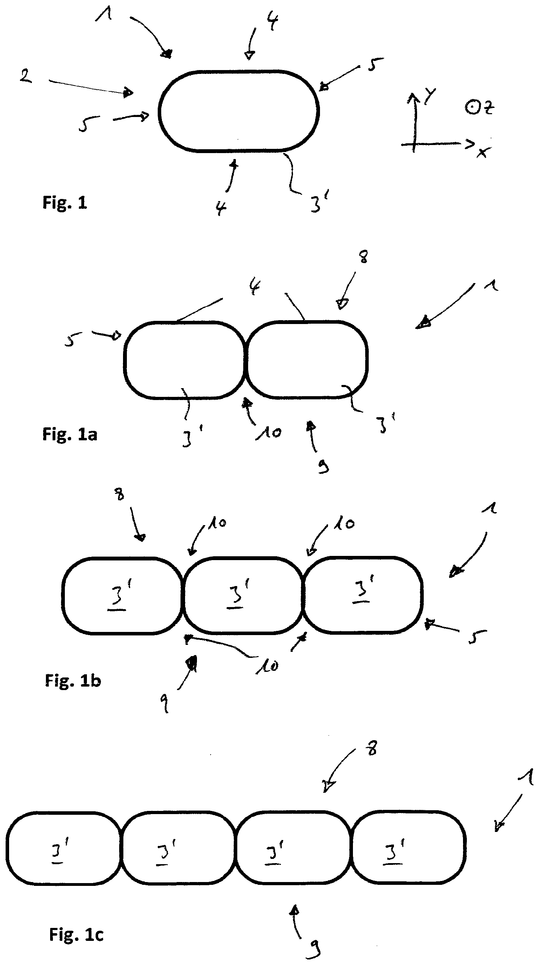

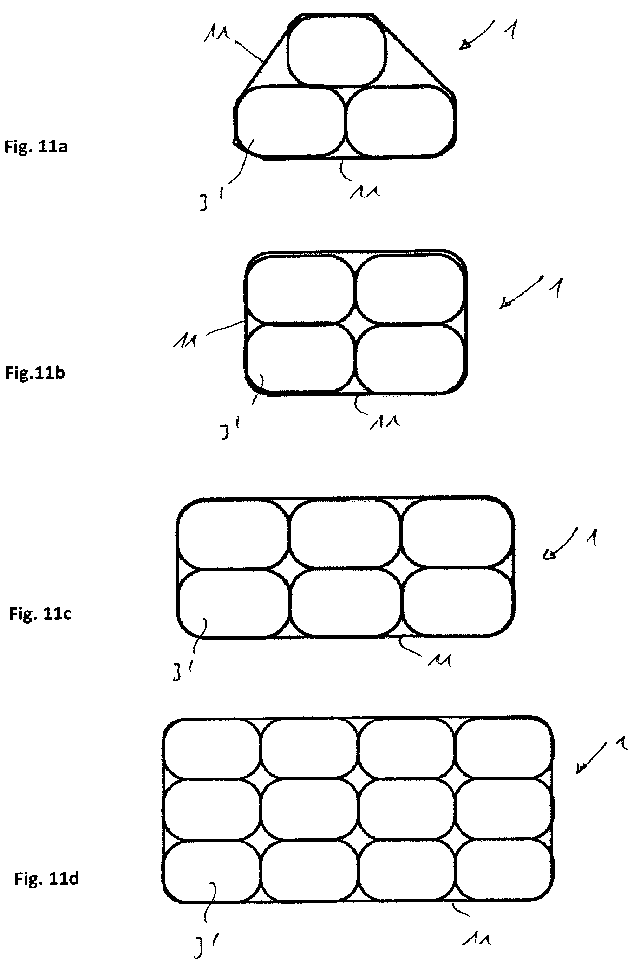

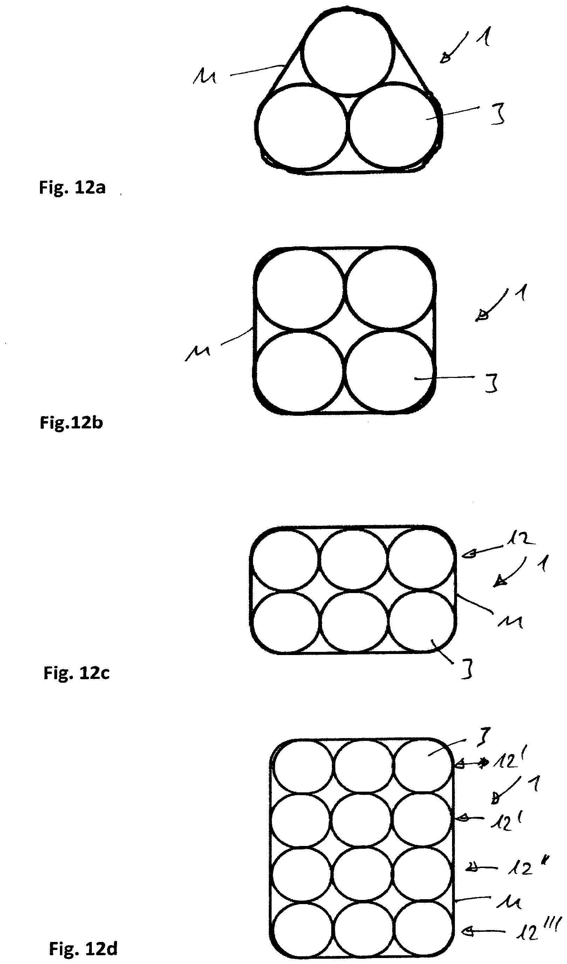

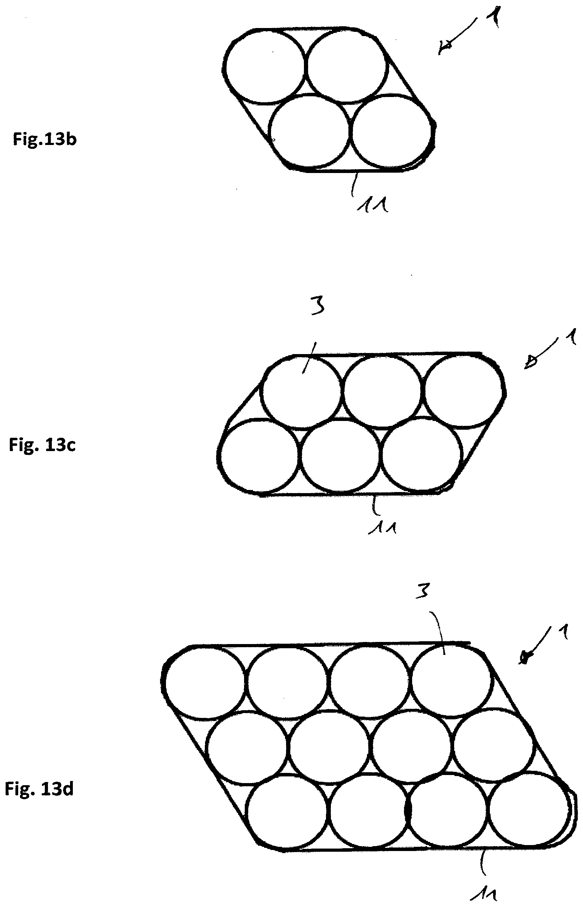

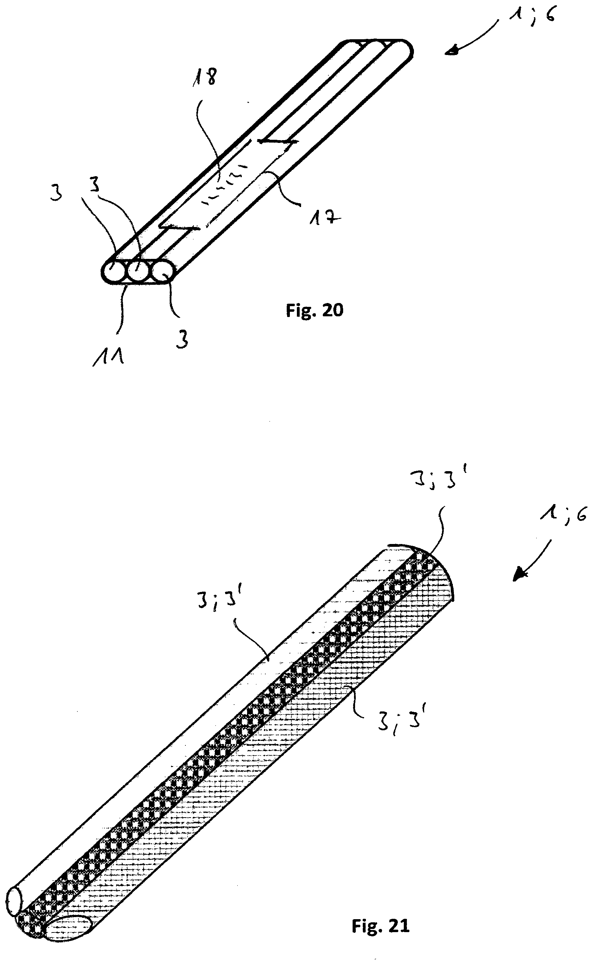

Die Figuren 1 bis 22b zeigen schematische Darstellungen. Der Fig. 1 kann ein Papierprofil 1 in seitlicher Ansicht entnommen werden. Zu sehen sind in Fig. 1 die Schmalseite 2 des Papierprofils 1. Das Papierprofil 1 gemäß Fig. 1 ist aus einem soliden Papierstab 3' gebildet, welcher an zwei gegenüberliegenden Längsstellen 4 eines ansonsten zylindrisch geformten Stabs 3' zusammengedrückt oder flachgepresst ist. Jeder der in den Figuren gezeigten Papierstäbe kann beispielsweise aus einer Papierbahn, etwa durch Wickeln, gebildet sein. Benachbart zu den Längsstellen 4 des Papierprofils respektive -stabs sind Rundbereiche 5, die auf die ursprünglich zylindrische Gestalt des Papierstabs 3' zurückgehen. Das Papierprofil 1 gemäß Fig. 1 hat entlang der durch die Rundbereiche 5 gedachten Achse (gemäß Fig. 1 die y-Achse) eine höhere Biegesteifigkeit als in der dazu senkrecht verlaufenden Achse (gemäß Fig. 1 die x-Achse). Insgesamt ergibt sich demnach in zumindest einer Biegerichtung eine höhere Biegesteifigkeit als bei dem zylindrischen Papierstab, aus dem das Papierprofil gemäß Fig. 1 gebildet ist.

Das Papierprofil 1 gemäß Fig. 1 kann beispielsweise auf eine Länge von etwa 76 mm bis etwa 113 mm abgelängt werden, und dann als Eisstiel 6 für ein Speiseeis 7 verwendet werden. Ein aus einem Profil 1 gemäß Fig. 1 gebildeter Eisstiel 6 bricht oder reißt auch bei starker mechanischer Beanspruchung nicht und splittert demnach nicht. Dadurch ist es ausgeschlossen, dass Splitter in das Speiseeis 7 oder in die Speiseeisverpackung gelangen.

Das Papierprofil 1 gemäß Fig. 1 kann eine Breite von etwa 10 mm aufweisen. Die Stärke des Profils 1 kann kleiner als 10 mm sein.

Die Figuren 1a bis 1c zeigen schematische Darstellungen von Varianten eines Profils 1, welches aus flachgepressten Papierstäben 3' gebildet ist. Der Fig. 1a kann ein Profil 1 umfassend zwei Papierstäbe 3' entnommen werden, gemäß Fig. 1b sind drei Papierstäbe 3' pro Profil 1 vorgesehen und gemäß Fig. 1c sind es vier Papierstäbe 3' pro Profil 1. Die Papierstäbe 3' sind gemäß der Figuren 1a, 1b und 1c nebeneinander in einer Ebene angeordnet. Hierdurch entsteht ein flaches Profil 1, wobei die Breite der Schmalseite 2 des Profils 1 durch die Anzahl der verarbeiteten Papierstäbe 3' bestimmbar ist. Die Papierstäbe 3' gemäß der Figuren 1a bis 1c können vor oder nach dem Anordnen durch Pressen abgeflacht worden sein. Es kann zweckmäßig sein, zunächst mehrere Papierstäbe parallel zueinander und benachbart anzuordnen, ggf. untereinander respektive miteinander zu verkleben, und anschließend den so gebildeten Papierstabverbund auf zumindest einer Seite flachzupressen.

Dadurch, dass mehrere Papierstäbe 3' vorgehen sind, erlangt das Profil 1 gemäß der Figuren 1a, 1b und 1c in zumindest einer Biegerichtung eine größere Biegesteifigkeit als ein einzelne Papierstab 3 aufweist.

Den Figuren 1a, 1b und 1c kann entnommen werden, dass das Profil 1 an den gegenüberliegenden, Seiten 8, 9 eine oder mehrere Rillen 10 aufweist. Die Rillen 10 verlaufen parallel zur Längsrichtung des Profils 1 und sie verändern die Haptik des Profils 1. Gleichzeitig eignen sich die Rillen 10 dazu, das Profil 1 von anderen Profilen zu unterscheiden, also als Wiedererkennungsmerkmal. Schließlich können sich die Rillen 10 bei der Verwendung des Profils 1 als Verpackungselement oder Verpackungsinstrument dazu eignen, mit dem zu Verpackenden, also beispielsweise mit einem Lebensmittel wie Speiseeis, besser zu verkrallen. Wird das Profil 1 beispielsweise für einen Eisstiel verwendet, wird das Eis an Profil 1, welches Rillen 10, umfasst, besser, insbesondere länger, haften, was ein Anwender (Verbraucher) insofern zu schätzen weiß, als sich die Eiscreme nicht oder zumindest mit geringerer Wahrscheinlichkeit in ungewünschter Weise von dem Stiel löst und herunterfällt.

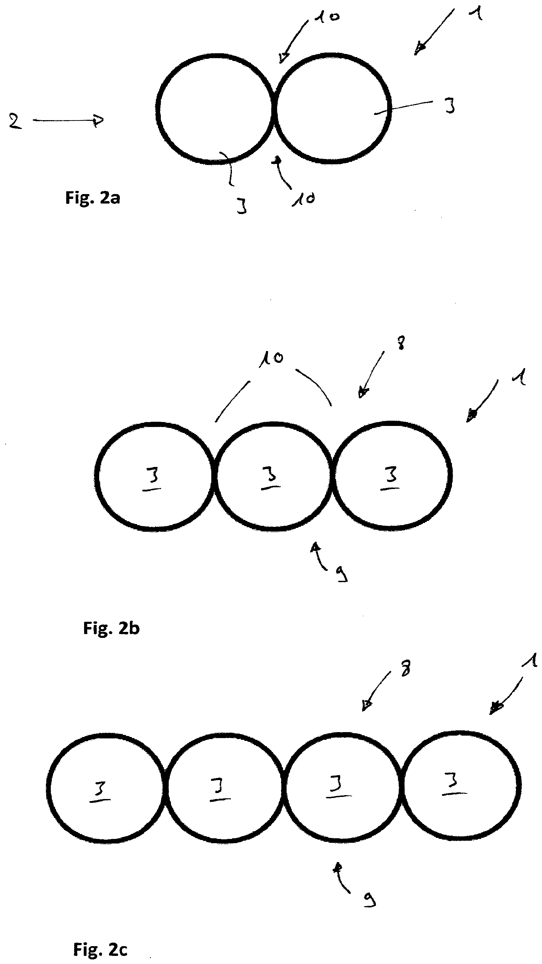

Die Figuren 2a bis 2c zeigen ein Papierprofil 1, welches aus mehreren, nämlich aus zwei, drei oder vier, Papierstäben 3 gebildet ist. Die Papierstäbe 3 haben jeweils einen (kreisrunden oder nahezu (kreis-)runden Querschnitt, sind demnach anders als in den Figuren 1a bis 1c gemäß der Figuren 2a bis 2c nicht flachgepresst. Die Papierstäbe 3 gemäß der in den Figuren 2a bis 2c dargestellten Profilen 1 können durch Kleben, d. h. mittels Klebstoff, Leim oder Haftmittel, miteinander verbunden sein. Es kann auch eine andere Verbindung vorgesehen sein, etwa durch Einsatz thermischer oder mechanischer Mittel. Im Bereich der Kontaktstellen 11 der Stäbe 3 sind eine Rille 10 (Fig. 2a) oder mehrere Rillen 10 (Figuren 2b und 2c) angeordnet. Für die Rillen 10 gilt das weiter oben Beschriebene entsprechend.

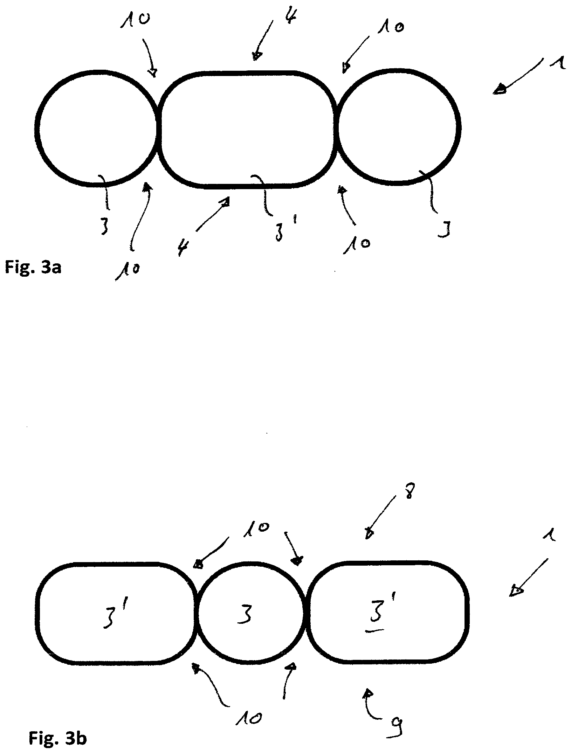

Die Figuren 3a und 3b zeigen schematisch jeweils ein Profil 1, welches jeweils aus drei Papierstäben 3, 3' gebildet ist. Die Papierstäbe 3, 3' sind in einer Ebene nebeneinander und parallel zueinander angeordnet. Die Papierstäbe 3, 3' eines der Profile 1 gemäß der Figuren 3a und 3b unterscheiden sich hinsichtlich ihrer Form. Gemäß Fig. 3a umfasst das Profil 1 zwei runde oder nahezu runde Stäbe 3, zwischen denen ein weiterer, flachgepresster Stab 3' angeordnet ist. Das Flachpressen des Stabs 3' kann vor oder nach dem Anordnen der drei das Profil 1 bildenden Stäbe 3, 3' erfolgen.

Gemäß Fig. 3b ist der mittlere Stab 3 rund oder nahezu rund, d. h. nicht flachgepresst, während die äußeren Stäbe 3' flachgepresst sind. Hinsichtlich der Herstellung des Profils 1 gemäß der Fig. 3a oder 3b kann es zweckmäßig sein, runde oder nahezu runde Stäbe mit großem Durchmesser (Stab 3') und kleinem Durchmesser (Stab 3) abwechselnd nebeneinander anzuordnen und diese Anordnung anschließend flachzupressen, so dass die Stärke (Dicke) des Profils 1 etwa dem kleineren Durchmesser der beteiligten Stäbe entspricht, also dem Durchmesser der runden Stäbe 3.

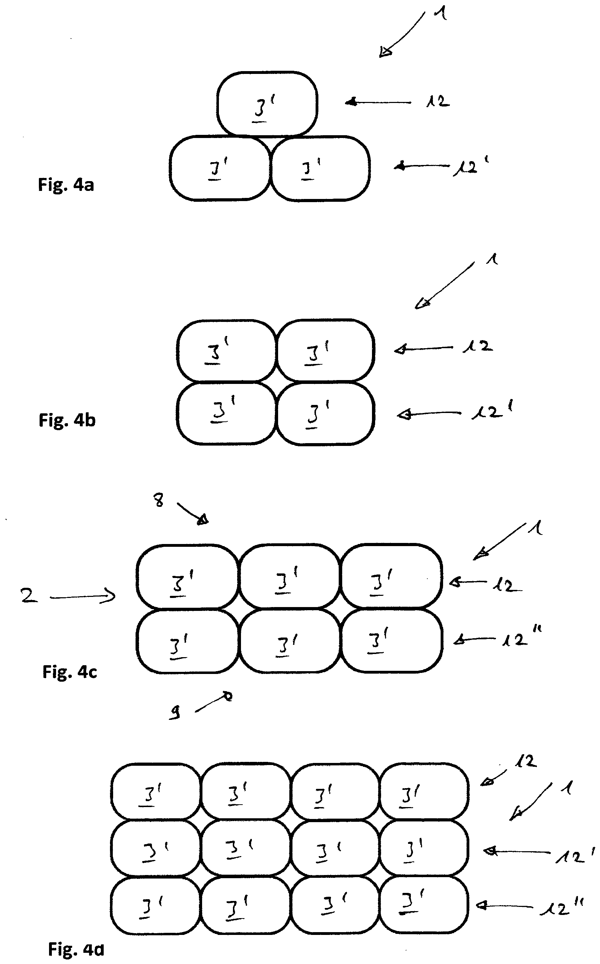

Die Figuren 4a bis 4d, 5a bis 5d, 6b bis 6d und 7 zeigen unterschiedliche Varianten von Profilen 1, die jeweils mehrlagig gebildet sind, d. h. bei denen eine erste Lage 12 Papierstäbe 3 vorgesehen ist und zumindest eine darauf angeordnete zweite Papierstab-Lage 12'. Es können auch drei, vier oder mehr Lagen (12", ...) vorgesehen sein. Durch die mehrlagige Anordnung der Papierstäbe 3 wird eine weitere Verbesserung der Biegesteifigkeit des Profils 1 erzielt. Insbesondere bei starker Biegebelastung des Profils 1 kann es zu strukturellen Veränderungen zwischen den Lagen (12, 12', 12") kommen, wodurch die Papierstäbe selbst weniger beansprucht werden und es nicht so schnell zu einem Riss respektive Bruch des Profils 1 kommen kann.

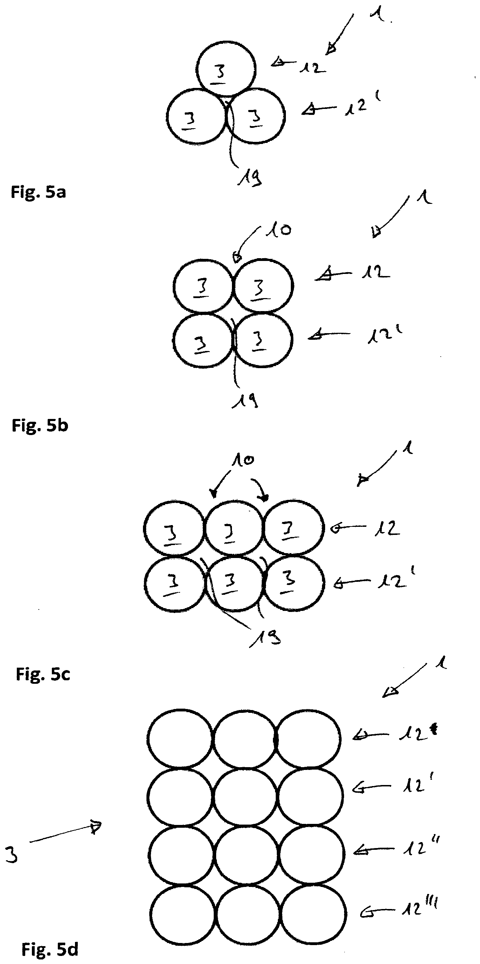

Fig. 4a zeigt ein zweilagiges Profil 1 mit einer aus zwei flachgedrückten Papierstäben 3 gebildeten ersten Lage 12. Auf der ersten Lage 12 ist eine zweite Lage 12' angeordnet, die aus einem flachgedrückten Papierstab 3 gebildet ist. Eine Profil-Variante mit ähnlicher Stapelkonfiguration, jedoch mit runden, also nicht-flachgedrückten, Papierstäben 3 anstelle der flachgedrückten Stäbe 3' zeigt die Fig. 5a.

Die Figuren 4b und 5b zeigen Profil-Varianten, bei denen die erste und die zweite Lage (12, 12') jeweils aus zwei Papierstäben gebildet sind. Gemäß der Figuren 4c und 5c umfasst jede Lage 12, 12' drei Papierstäbe und gemäß Figuren 4d und 5d sind pro Lage vier Papierstäbe vorgesehen. Es können weitere Varianten vorgesehen sein, die mehr als vier Stäbe pro Lage (12, 12', ...) umfassen. Es können ferner weitere Profil-Varianten vorgehen sein, die mehr als zwei Papierstab-Lagen umfassen, wobei jede Lage (12, 12', 12", ...) beliebig viele Stäbe 3, 3' umfassen kann. Insofern sind zahlreiche Stapelkonfigurationen in zahlreichen Profil-Varianten möglich. Durch Variation der Anzahl der Lagen und/oder der Anzahl der Stäbe pro Lage können folgende Profileigenschaften beeinflusst werden: die Profil-Breite (Anzahl der Stäbe pro Lage), die Profil-Stärke (Anzahl der Lagen), sowie die mechanischen, insbesondere biegemechanischen, Eigenschaften des Profils (Lagen- und/oder Stabzahl). Durch Variation der Anzahl der Lagen/Stäbe kann das Profil 1 demnach an die an das Profil 1 gerichteten Anforderungen angepasst werden. Soll aus dem Profil 1 beispielsweise ein Speiseeisstiel 6 gebildet werden, können wenige Lagen (12, 12') und pro Lage wenige Stäbe 3 ausreichend sein (beispielsweise eine oder zwei Lagen mit jeweils bis zu fünf Stäben). Soll aus dem Profil 1 hingegen eine biegesteife Spannvorrichtung, etwa ein Schuhspanner, gebildet werden, so wäre eine größere Anzahl von Lagen und/oder Stäben pro Lage zweckmäßig (beispielsweise fünf bis zehn Lagen mit jeweils fünf bis zehn Stäben).

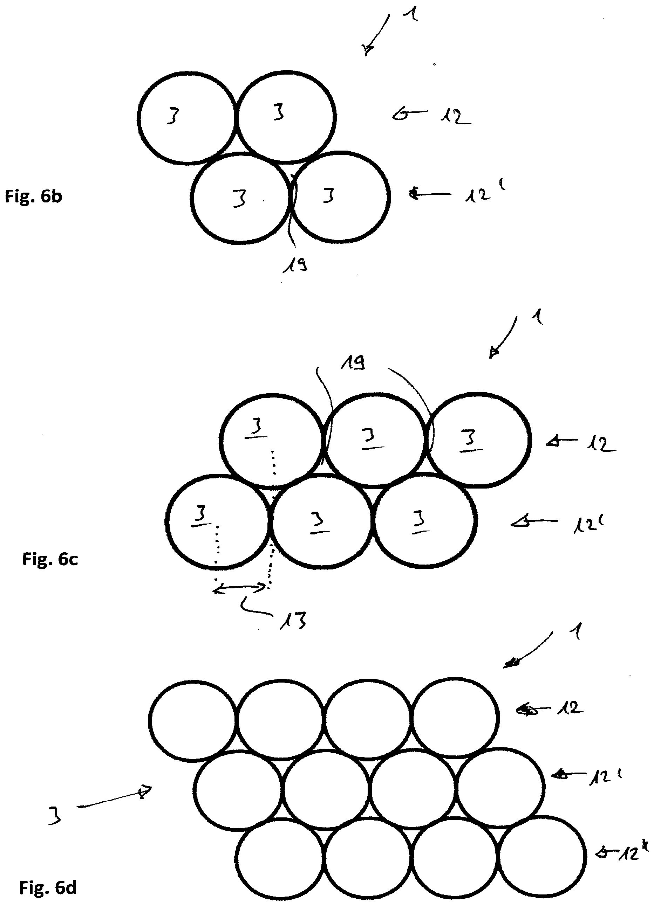

Den Figuren 6b bis 6d können Profil-Varianten entnommen werden, bei denen die Stäbe der zweiten Lage 12' in der Rille 10 respektive in den Rillen 10 zwischen den Stäben 3 der ersten Lage 12 angeordnet sind. Insofern umfassen die Stapelkonfigurationen gemäß der Figuren 6b bis 6d einen seitlichen Versatz 13 benachbarter Lagen. Gemäß der (hier exemplarisch dargestellten) Stapelkonfigurationen gemäß der Figuren 6b bis 6d wird das Volumen der Zwischenräume 19 zwischen den Stäben 3, 3' reduziert, wodurch eine höhere Dichte und Festigkeit des Profils 1 erzielbar wird. Bei einer hohen Festigkeitsanforderung wäre die Stapelkonfiguration gemäß Figuren 6b bis 6d zu bevorzugen, bei hoher Biegebeanspruchbarkeit könnten die Profilkonfigurationen gemäß der Figuren 4a bis 4d respektive 5a bis 5d bevorzugt werden.

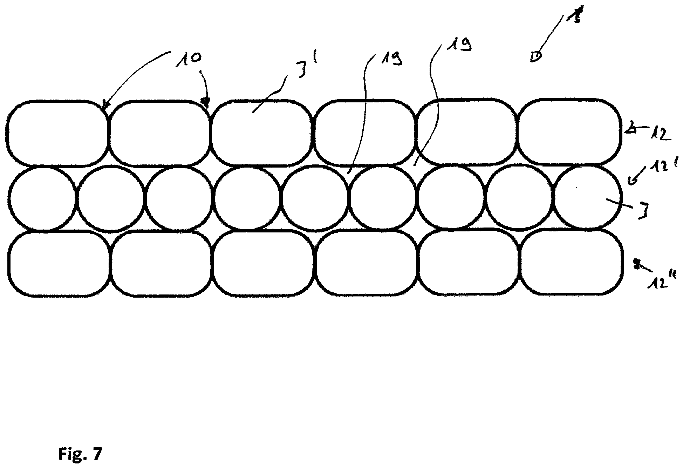

Eine Profilkonfiguration mit drei Lagen 12, 12', 12" und umfassend Stäbe 3 mit einem ersten Durchmesser sowie flachgedrückte Stäbe 3' kann der Fig. 7 entnommen werden. Die flachgedrückten Stäbe 3' sind aus ursprünglich runden Stäben durch Flachpressen gebildet, wobei die ursprünglich runden Stäbe einen zweiten Durchmesser haben, der größer war als der erste Durchmesser der runden Stäbe 3. Gemäß Fig. 7 ist die mittlere Lage (12') aus runden Stäben 3 gebildet, die angrenzenden äußeren Lagen (12, 12") sind aus flachgepressten Stäben 3' gebildet. Alternativ kann eine Variante vorgesehen sein, bei der die runden Stäbe 3 die äußeren Lagen bilden und die flachgepressten Stäbe die mittlere Lage. Bei mehr als drei Lagen können alternierende oder sich wiederholende Konfigurationen vorgesehen sein (ABABAB, ABBABBABBA, ABBAABBAABBA, u. v. m., wobei A, B hier Variablen für unterschiedlich gebildete Lagen sind).

Bei allen Konfigurationen gemäß der Figuren 1a bis 1c, 2a bis 2c, 3a, 3b, 4a bis 4d, 5a bis 5d, 6b bis 6d und bei Fig. 7 kann vorgesehen sein, dass die Stäbe 3, 3' miteinander respektive untereinander verklebt oder verleimt sind oder in sonstiger Weise aneinander haften und insofern einen (Haft- oder Leim-)Verbund bilden. Alternativ dazu kann vorgesehen sein, dass anstelle eines Haftmittels (Klebstoff, Leim) ein Mantelelement 11 vorgehen ist, das die (zumindest äußeren) Stäbe 3, 3' des Profils 1 zumindest abschnittsweise umschließt. Es können auch mehrere Mantelelemente 11 vorgesehen sein, die entlang der Längsrichtung des Profils 1 angeordnet sind, und die einen stabilen Zusammenschluss der Stäbe (3, 3') des Profils 1 erzeugen.

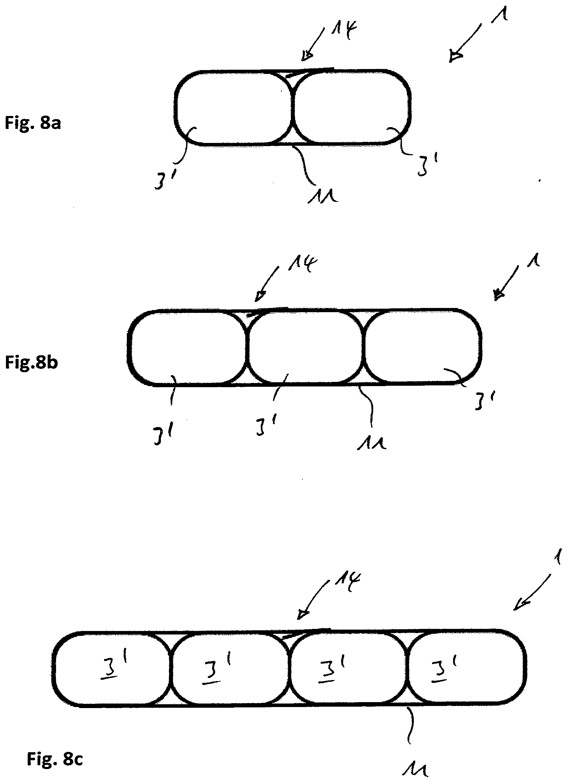

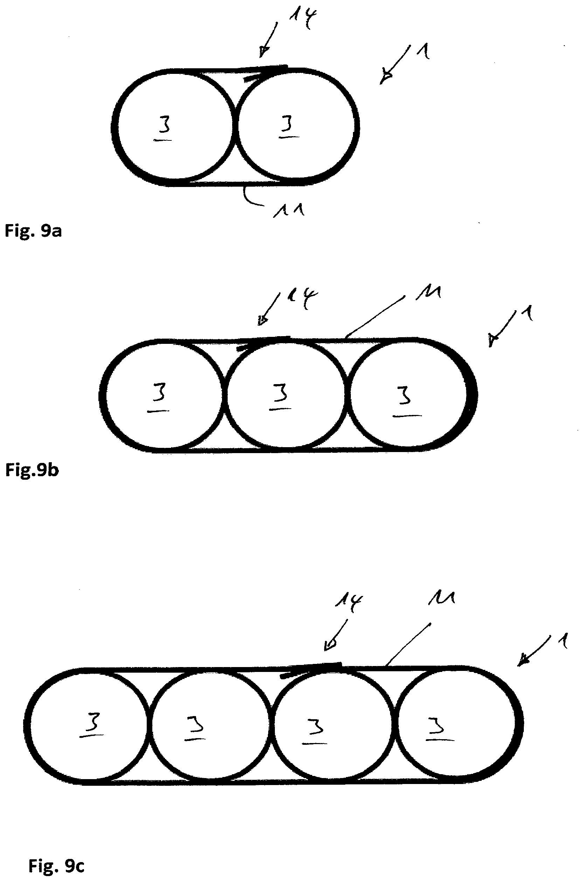

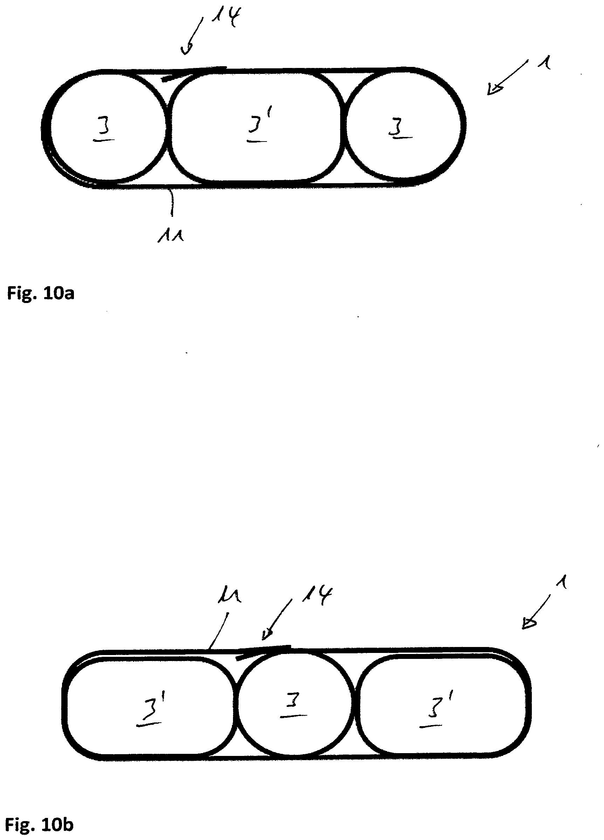

Analog zu den oben beschriebenen Stapelkonfigurationen unterschiedlicher Profil-Varianten zeigen die Figuren 8a bis 8c, 9a bis 9c, 10a, 10b, 11a bis 11d, 12a bis 12d, 13b bis 13d sowie Fig. 14 Konfigurationen, bei denen die Stäbe respektive die äußeren Stäbe mit einer Banderole 11 umwickelt sind. Banderole oder Mantelelement 11 können aus einem Papier, insbesondere aus einem um die Stäbe gewickelten Papierstreifen, gebildet sein. Der Papiermantel 11 kann aus einem mehrlagigen Papiermaterial gebildet sein. Die Endbereiche 14 des Mantelelements 11 können sich zumindest abschnittsweise überlappen, wie etwa in den Figuren 8a bis 8c, und 9a bis 9c sowie in den Figuren 10a und 10b schematisch dargestellt. Die überlappenden Endbereiche 14 können miteinander verklebt oder verleimt sein, es kann auch eine mechanische Verbindung der Endbereiche 14 vorgesehen sein, etwa eine Falt- und/oder Knickstelle respektive Falz. Es kann auch eine Verbindung der Endbereiche 14 durch thermischen Energieeintrag, etwa mit einer Schweißnaht, vorgesehen sein. Es kann auch vorgesehen sein, dass die Endbereiche 14 des Mantelstreifens aneinander stoßen (Auf-Stoß-Anordnung). Es kann zum Verbinden der Endbereiche 14 des Mantels 11 auch ein Verbindungelement, etwa ein Klebestreifen, vorgesehen sein.

Durch das Umwickeln der Stäbe 3, 3' mit einem Mantelelement 11 erhält das Profil 1 eine im Wesentlichen glatte Mantelfläche. Das Mantelelement 11 kann farbig sein und/oder mit einer Beschriftung und/oder mit einem Motiv und/oder mit einem Logo bedruckbar, wodurch das Profil 1 kostengünstiger gestaltbar ist. Im Übrigen wird durch das Mantelelement 11 eine hochwertigere Haptik des Profils 1 erreicht, der Verwender des Profils 1 kann nicht unmittelbar auf die Stäbe 3, 3' schließen und hat den Eindruck, ein kompaktes, stabiles, leichtes und umweltschonendes Papierprofil in den Händen zu halten. Diese Eigenschaft des Profils ist besonders dann gewünscht, wenn das Profil 1 im Zusammenhang mit Verpackungskomponenten oder Verpackungsausrüstungen verwendet wird.

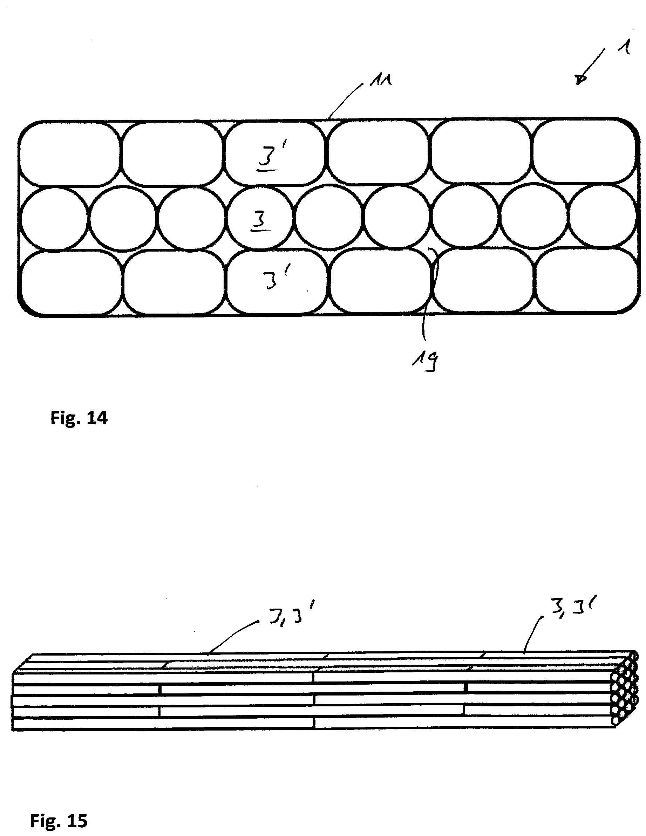

Einen Papierstabverbund 15 aus einer Vielzahl angeordneter Papierstäbe 3 kann der Fig. 15 entnommen werden. Die Papierstäbe 3, 3' sind nicht nur nebeneinander angeordnet sondern auch hintereinander. So kann ein Profil 1 beliebiger Ausdehnung in z-Richtung (Länge) gebildet werden. Insbesondere kann ein Profil 1 mit einer Profillänge von beispielsweise ca. 1 m oder länger hergestellt werden, welches aus einer Vielzahl von Stäben 3, 3' mit einer Stablänge von beispielsweise ca. 20 cm oder weniger gebildet ist.

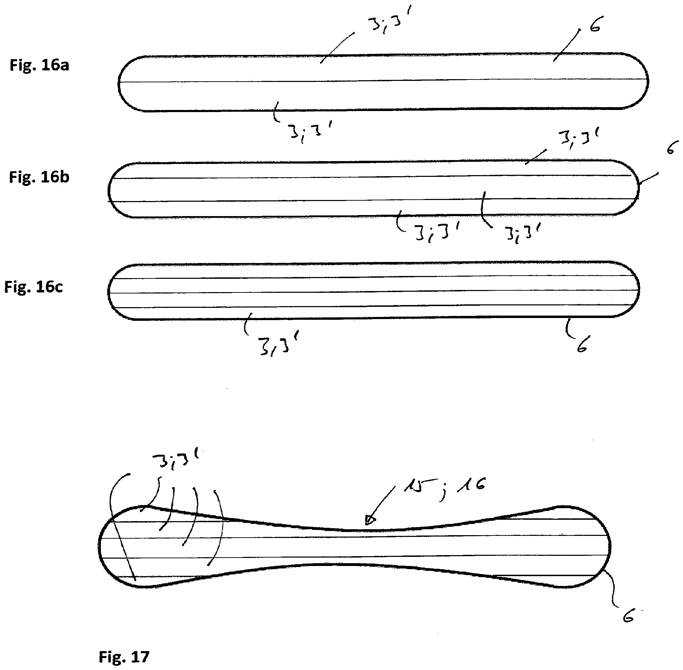

Verwendungsbeispiele des hier beschriebenen Papierprofils 1 können den Figuren 16a bis 16c, 17, 18a, 18b, 19a, 19b, 20 und 21 entnommen werden. Die Figuren 16a bis 16c zeigen jeweils erfindungsgemäße Eisstiele 6 für Speiseeis 7. Die Eisstiele 6 sind eine Verpackungsausrüstung einer Speiseeisverpackung. Das Speiseeis 7 wird auf dem Eisstiel 6 angeordnet. Eis mit Eisstiel, also das Eis-am-Stiel, wird in einer Umverpackung aus Papier oder Karton angeordnet. Der Verbraucher entnimmt üblicherweise den Eisstiel 6 samt Eis 7 aus der Umverpackung und verzehrt das Eis 7, wobei er den Eisstiel 6 greift oder hält. Fig. 6a zeigt einen Eisstiel 6 der aus einem Papierprofil 1 gebildet ist, welches wiederum aus zumindest einer Papierstablage 12 mit zwei benachbarten Papierstäben 3, 3' gebildet ist. Die Papierstäbe 3, 3' können rund sein oder flachgedrückt. Die Stäbe 3, 3' können aneinandergeklebt sein oder mit einem Mantelelement 11 umwickelt sein. Das Profil 1 wird auf die Länge des Eisstiels 6 abgelängt, etwa durch Schneiden oder Stanzen, ggf. an den Enden abgerundet oder entsprechend geschnitten.

Gemäß Fig. 16b ist ein Profil mit drei Stäben (3, 3') zur Herstellung des Eisstiels 6 verwendet worden, gemäß Fig. 16c sind es vier Stäbe (3, 3'). Die Profile 1 zur Herstellung der Eisstiele 6 gemäß der Figuren 16a bis 16c haben alle in etwa die gleiche Profilbreite, lediglich die Breite (der Durchmesser) der Papierstäbe (3, 3') variiert.

Der Fig. 17 kann ein Eisstiel 6 entnommen werden, der aus einem Profil 1 gebildet wurde, welches eine Kontur 15 aufweist. Die Kontur 15 kann durch Schneiden oder Stanzen in dem Profil 1 angeordnet werden. Der Eisstiel 6 gemäß der Fig. 17 hat entlang der Längsrichtung eine Taille 16, wodurch der Stiel 6 die Gestalt eines Paddels respektive Doppelpaddels aufweist. Der paddelförmige Stiel liegt besser in der Hand, der Verbraucher kann das Stieleis besser und sicherer festhalten. Gleichzeitig wird im Bereich der Taille 16 Papiermaterial eingespart, was ökologische Vorteile bietet. Außerdem wird der Stiel durch die Kontur 15 leichter, was den Aufwand für Transport und Lagerhaltung bei großen Stückzahlen reduziert.

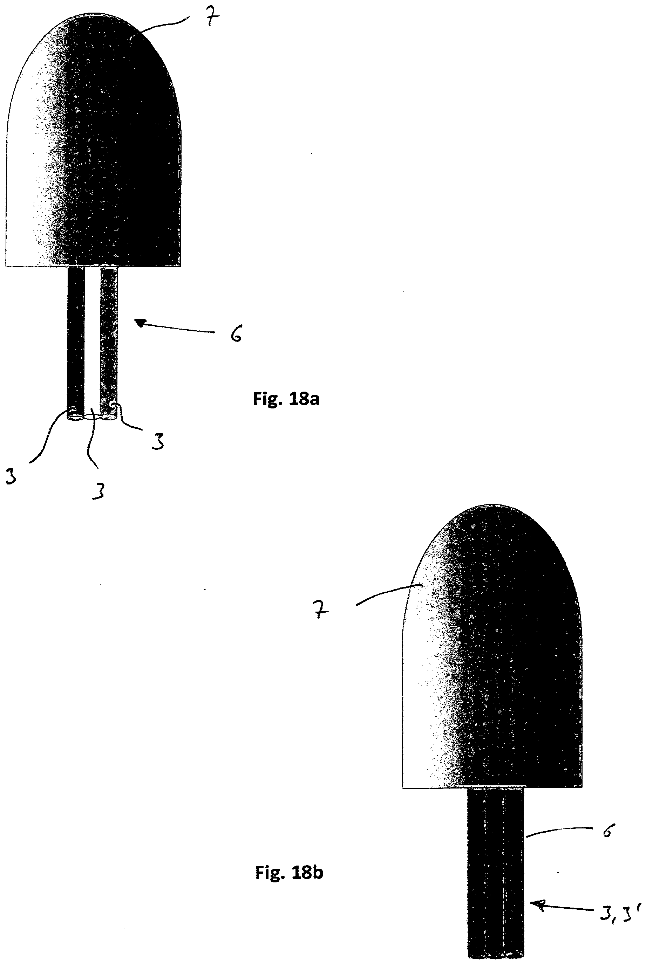

Ein Speiseeis 7 mit in dem Speiseeis 7 angeordneten Eisstielen 6 kann den Figuren 18a und 18b schematisch entnommen werden. Der Eisstiel 6 gemäß Fig. 18b ist aus einem Profil 1 gebildet, welches drei (gleich-)farbige Papierstäbe 3 umfasst. Der Eisstiel 6 gemäß Fig. 18a ist aus einem Profil 1 gebildet, welches drei Papierstäbe 3 umfasst, die sich jeweils in ihrer Farbe unterscheiden. So kann der Eisstiel in den Farben gemäß der Marke des Eisherstellers hergestellt sein, oder es können Farben verwendet werden, die auf eine nationale oder regionale Herkunft schließen lassen. Ein Eisstiel 6 mit den Papierstabfarben schwarz, rot und gelb, ließe beispielsweise auf ein Eis mit deutscher Herkunft schließen, ein Eisstiel 6 mit den Farben blau, weiß, rot auf ein Eis 7 französischer Herkunft oder Geschmackstype und ein Eisstiel 6 in den Farben grün, weiß, rot ließe auf das Herkunftsland Italien schließen. Beliebig viele andere Farbkombinationen sind möglich. Die Herstellung eines bunten Profils 1 erfolgt dadurch, dass farbige Papierstäbe 3, 3' verwendet werden. Alternativ kann Farbe oder Aussehen des Profils durch einen farbigen respektive gestalteten Mantel 11 erreicht werden.

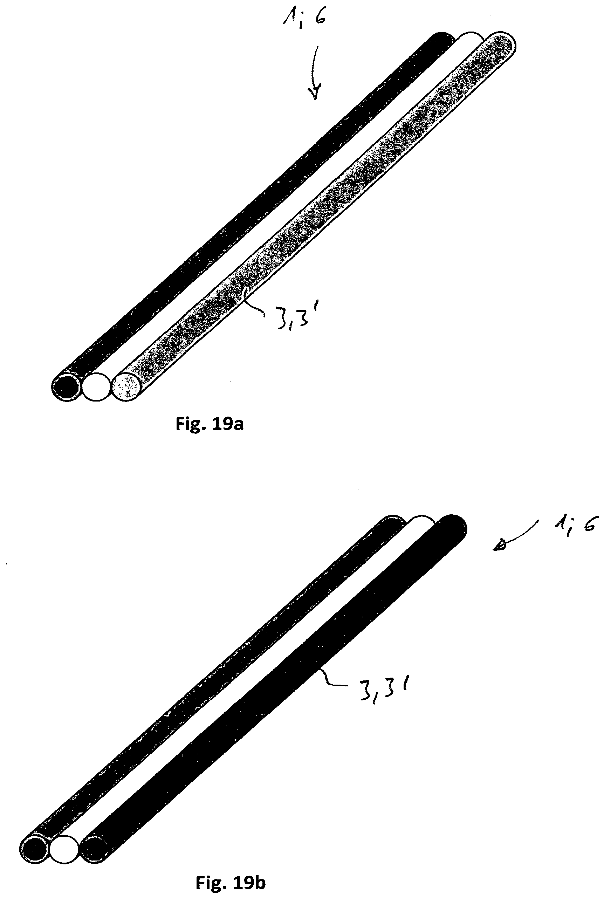

Den Figuren 19a, 19b, 20 und 21 können verschiedene Profilvarianten mit unterschiedlichen Gestaltungseigenschaften entnommen werden. Durch die Auswahl der verwendeten Papierstäbe 3, 3' können viele unterschiedliche Gestaltungsvarianten ermöglicht werden. Eine weitere Gestaltungsmaßnahme besteht in dem Anordnen eines Kennzeichnungsbereichs 17 auf dem Profil 1. Der Kennzeichnungsbereich 17 kann auf den Stäben 3, 3' oder auf einem Mantelelement 11 angeordnet sein. Der Kennzeichnungsbereich 17 kann aufgedruckt oder aufgeprägt sein. In dem Kennzeichnungsbereich 16 können eine Beschriftung 18 und/oder ein Logo und/oder ein (Bild-)Motiv angeordnet sein.

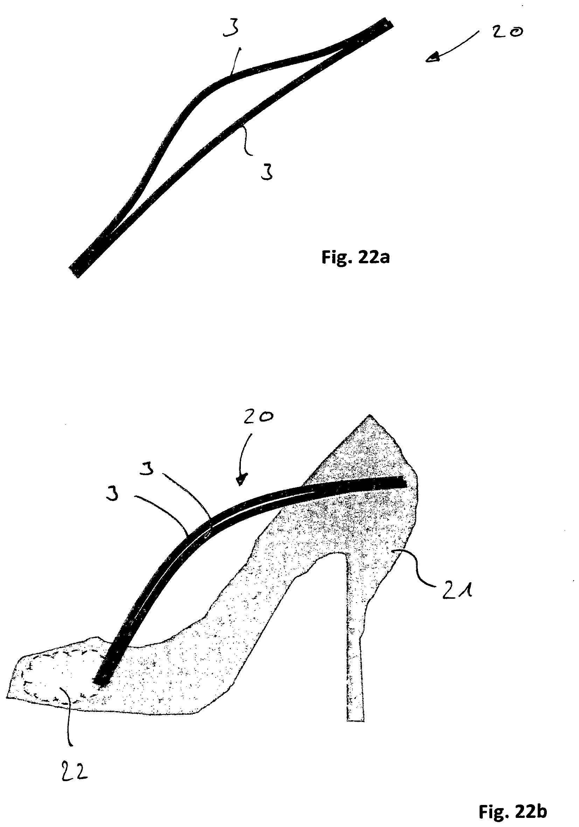

Eine schematische seitliche Ansicht eines nicht erfindungsgemäßen Schuhspanners 20 kann der Fig. 22a entnommen werden. Der Schuhspanner 20 umfasst einen ersten Papierstab 3 sowie einen zweiten Papierstab 3. Beide Papierstäbe 3 sind endseitig miteinander verbunden, wobei der erste Papierstab 3 etwas länger ist als der zweite Papierstab 3. Durch den Längenunterschied beider Papierstäbe 3 und dadurch, dass eine Verbindung der Papierstäbe 3 an den jeweiligen Stabenden vorgesehen ist, weist der längere Stab 3 eine Biegung auf, wodurch die Spannvorrichtung 20 eine mechanische Vorspannung hat.

Gemäß der schematischen Darstellung der Fig. 22b ist der Schuhspanner gemäß Fig. 22a in einem Damenschuh 21 eingesetzt. Im vorderen Bereich des Damenschuhs 21 ist ein Stopfpapier angeordnet, gegen welches der Schuhspanner 20 drückt. Der Schuhspanner 20 ist gegenüber der in Fig. 22a dargestellten, ungebogenen Ausgangsstellung nunmehr mechanisch verbogen, wodurch der erste und der zweite Papierstab aufeinander Kräfte übertragen. Das Verbiegen der Papierstäbe 3 des Spanners 20 erfolgt reversibel, d. h. die Stäbe 3 sind ohne Materialschäden wieder in die Ausgangsstellung (Fig. 22a) bringbar. Aufgrund der Vorspannung (Fig. 22a) "möchte" der Schuhspanner in seine Ausgangsform (Fig. 22a) zurückkehren, wodurch der Schuhspanner 20 auf den Schuh 21 eine den Schuh 21 spannende resultierende Kraft überträgt.

Der Schuhspanner 20 gemäß den Figuren 22a und 22b ist aus Papierstäben gebildet, d. h. aus einem Papiermaterial. Auch das Stopfpapier 22 ist aus einem Papiermaterial gebildet. Wird der Schuh 21 mit Schuhspanner 20 und Stopfpapier 22 nun in einem (Schuh-)Karton (um-)verpackt, so kann die gesamte Schuhverpackung (Karton + Stopfpapier 22 + Schuhspanner 20) der Papiermülltonne zugeführt werden. Eine Mülltrennung der Schuhverpackung entfällt, da außer Papiermaterial kein weiteres Verpackungsmaterial erforderlich ist.

### BEZUGSZEICHENLISTE

- 1: Papierprofil
- 2: Schmalseite
- 3,3': Papierstab
- 4: Längsstellen
- 5: Rundbereich
- 6: Eisstiel
- 7: Speiseeis
- 8: Seite
- 9: Seite
- 10: Rille
- 11: Mantelelement
- 12, 12', 12": Lage
- 13: Versatz
- 14: Endbereich
- 15: Kontur
- 16: Taille
- 17: Kennzeichnungsbereich
- 18: Beschriftung
- 19: Zwischenraum
- 20: Schuhspanner
- 21: Schuh
- 22: Stopfpapier

## Patentansprüche

1. Eisstiel (6) für Speiseeis, gebildet aus einem soliden Papierprofil (1) wobei das Papierprofil (1) eine längliche Form aufweist, wobei das Papierprofil (1) aus mehreren runden oder nahezu runden biegesteifen, soliden Papierstäben (3) gebildet ist, wodurch die Biegesteifigkeit des Papierprofils (1) in zumindest einer Biegerichtung zumindest der Biegesteifigkeit eines der biegesteifen Papierstäbe (3) entspricht, wobei durch Biegen des Papierprofils (1) zumindest bereichsweise eine rückstellende, der Biegerichtung entgegenwirkende, Kraft erzeugbar ist, **dadurch geke nnzeichnet,** dass jeder Papierstab aus einer Papierbahn gefertigt ist, dass die Papierstäbe (3, 3') identischen oder nahezu identischen Durchmesser haben, und dass die Biegesteifigkeit des Papierprofils (1) die Biegesteifigkeit eines der Papierstäbe (3) in zumindest einer Biegerichtung übertrifft.

2. Eisstiel (6) nach Anspruch 1, **dadurch gekennzeichnet, dass** zumindest einer der Papierstäbe (3) durch Flachdrücken bearbeitet ist, wodurch das Papierprofil (1) flach ist.

3. Eisstiel (6) nach Anspruch 1 oder 2, **dadurch gekennzeichnet, dass** das Profil (1) aus zumindest einer Lage Papierstäbe (3, 3') gebildet ist, in der zumindest zwei Papierstäbe (3, 3') nebeneinander und zueinander parallel angeordnet sind, wobei vorzugsweise zumindest ein Papierstab (3, 3') zumindest abschnittsweise eine an zumindest einer Längsseite oder an zumindest einer Schmalseite angeordnete Rundung und/oder Schräge umfasst, so dass das Papierprofil (1) eine Kontur (15) aufweist.

4. Eisstiel (6) nach einem der Ansprüche 1 bis3, **dadurch gekennzeichnet, dass** sich zumindest ein Papierstab (3, 3') in einer Eigenschaft von zumindest einem anderen Papierstab (3, 3') unterscheidet, wobei die Eigenschaft Form, Rundung, Farbe, Oberflächenbeschaffenheit, Material und/oder Biegesteifigkeit umfassen kann.

5. Eisstiel (6) nach einem der Ansprüche 1 bis 4, **dadurch gekennzeichnet, dass** zumindest einige der Papierstäbe (3, 3') des Papierprofils (1), zumindest abschnittsweise, insbesondere durch Haften oder Kleben, untereinander und/oder miteinander verbunden sind.

6. Eisstiel (6) nach einem der Ansprüche 1 bis 5, **dadurch gekennzeichnet, dass** die Papierstäbe (3, 3') entlang des Umfangs des Papierprofils (1) durch zumindest ein aus Papier gebildetes Mantelelement (11) miteinander verbunden sind.

7. Eisstiel (6) nach einem der Ansprüche 1 bis 6, **gekennzeichnet durch** einen auf dem Papierprofil (1) angeordneten Kennzeichnungsbereich (17), auf dem eine Kennzeichnung und/oder ein Logo anordbar ist.

## Claims

1. An ice cream stick (6) for ice cream, formed from a solid paper profile (1), wherein the paper profile (1) has an elongated shape, wherein the paper profile (1) is formed from a plurality of round or almost round flexurally rigid, solid paper rods (3), whereby the flexural rigidity of the paper profile (1) corresponds in at least one bending direction at least to the flexural rigidity of one of the flexurally rigid paper rods (3), wherein by bending the paper profile (1) at least in sections a restoring force counteracting the bending direction is able to be generated, **characterized in that** each paper rod is manufactured from a paper web, that the paper rods (3, 3') have an identical or almost identical diameter, and that the flexural rigidity of the paper profile (1) exceeds the flexural rigidity of one of the paper rods (3) in at least one bending direction.

2. The ice cream stick (6) according to Claim 1, **characterized in that** at least one of the paper rods (3) is processed by flattening, whereby the paper profile (1) is flat.

3. The ice cream stick (6) according to Claim 1 or 2, **characterized in that** the profile (1) is formed from at least one layer of paper rods (3, 3') in which at least two paper rods (3, 3') are arranged adjacent to one another and parallel to one another, wherein preferably at least one paper rod (3, 3') comprises, at least in sections, a curvature and/or slope arranged on at least one long side or on at least one narrow side, so that the paper profile (1) has a contour (15).

4. The ice cream stick (6) according to one of Claims 1 to 3, **characterized in that** at least one paper rod (3, 3') differs in a property from at least one other paper rod (3, 3'), wherein the property can comprise shape, curvature, colour, surface finish, material and/or flexural rigidity.

5. The ice cream stick (6) according to one of Claims 1 to 4, **characterized in that** at least some of the paper rods (3, 3') of the paper profile (1), at least in sections, are connected among one another and/or to one another in particular through adhesion or bonding.

6. The ice cream stick (6) according to one of Claims 1 to 5, **characterized in that** the paper rods (3, 3') are connected to one another along the circumference of the paper profile (1) by at least one shell element (11) formed from paper.

7. The ice cream stick (6) according to one of Claims 1 to 6, **characterized by** a marking region (17), arranged on the paper profile (1), on which a marking and/or a logo is able to be arranged.

## Revendications

1. Bâtonnet de glace (6) pour une glace alimentaire, formé d'un profilé de papier (1) solide, le profilé de papier (1) présentant une forme oblongue, le profilé de papier (1) étant formé de plusieurs baguettes de papier (3) rondes ou pratiquement rondes, rigides en flexion, la rigidité en flexion du profilé de papier (1) dans au moins une direction de flexion correspondant au moins à la rigidité en flexion de l'une des baguettes de papier (3) rigides en flexion, par la flexion du profilé de papier (1), au moins par endroits une force de rappel s'opposant au sens de flexion étant susceptible d'être générée, **caractérisé en ce que** chaque baguette de papier est fabriquée à partir d'une bande de papier, **en ce que** les baguettes de papier (3, 3') ont un diamètre identique ou pratiquement identique et **en ce que** la rigidité à la flexion du profilé de papier (1) est supérieure à la rigidité en flexion de l'une des baguettes de papier (3) dans au moins une direction de flexion.

2. Bâtonnet de glace (6) selon la revendication 1, **caractérisé en ce qu'**au moins l'une des baguettes de papier (3) est usinée par aplatissage, suite à quoi, le profilé de papier (1) est plat.

3. Bâtonnet de glace (6) selon la revendication 1 ou 2, **caractérisé en ce que** le profilé (1) est formé d'au moins une couche de baguettes de papier (3, 3'), dans laquelle au moins deux baguettes de papier (3, 3') sont placées côte à côte et à la parallèle l'une de l'autre, de préférence au moins une baguette de papier (3, 3') comprenant au moins par segments un arrondi et/ou une inclinaison placé (e) sur au moins un côté longitudinal ou sur au moins un côté étroit, de sorte que le profilé de papier (1) comporte un contour (15).

4. Bâtonnet de glace (6) selon l'une quelconque des revendications 1 à 3, **caractérisé en ce qu'**au moins une baguette de papier (3, 3') se différencie par une propriété d'au moins une autre baguette de papier (3, 3'), la propriété pouvant inclure la forme, l'arrondi, la teinte, la qualité de surface, la matière et/ou la rigidité en flexion.

5. Bâtonnet de glace (6) selon l'une quelconque des revendications 1 à 4, **caractérisé en ce qu'**au moins certaines des baguettes de papier (3, 3') du profilé de papier (1) sont assemblées ensemble et/ou les unes aux autres au moins par segments, notamment par adhérence ou par collage.

6. Bâtonnet de glace (6) selon l'une quelconque des revendications 1 à 5, **caractérisé en ce que** les baguettes de papier (3, 3') sont assemblées ensemble le long de la périphérie du profilé de papier (1) par au moins un élément d'enveloppe (11) formé en papier.

7. Bâtonnet de glace (6) selon l'une quelconque des revendications 1 à 6, **caractérisé par** une zone de marquage (17) placée sur le profilé de papier (1) sur laquelle peut être apposé un marquage et/ou un logo.
